(19) **Europäisches Patentamt / European Patent Office / Office européen des brevets**

(11) **EP 1 858 639 B1**

(12) **EUROPÄISCHE PATENTSCHRIFT**

(45) Veröffentlichungstag und Bekanntmachung des Hinweises auf die Patenterteilung:
**15.02.2012 Patentblatt 2012/07**

(21) Anmeldenummer: **06723513.5**

(22) Anmeldetag: **17.03.2006**

(51) Int Cl.:
***B01J 20/10*** *(2006.01)*

(86) Internationale Anmeldenummer:
**PCT/EP2006/002474**

(87) Internationale Veröffentlichungsnummer:
**WO 2006/097326 (21.09.2006 Gazette 2006/38)**

(54) **VERFAHREN ZUR ABTRENNUNG VON BIOMOLEKÜLEN AUS FLÜSSIGEN MEDIEN**

METHOD FOR SEPARATING BIOMOLECULES FROM LIQUID MEDIA

PROCÉDÉ POUR SÉPARER DES BIOMOLÉCULES DE MILIEUX LIQUIDES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **18.03.2005 DE 102005012639**

(43) Veröffentlichungstag der Anmeldung:
**28.11.2007 Patentblatt 2007/48**

(73) Patentinhaber: **SÜD-CHEMIE AG**
**80333 München (DE)**

(72) Erfinder: **SOHLING, Ulrich**
**85356 Freising (DE)**

(74) Vertreter: **Rembold, Hansjörg**
**Stolmár Scheele & Partner**
**Blumenstraße 17**
**80331 München (DE)**

(56) Entgegenhaltungen:
**WO-A-93/15832       WO-A-2004/103552**
**GB-A- 1 181 435      US-A- 4 717 699**

**Beschreibung**

Die Erfindung betrifft ein Verfahren zur Abtrennung von Biomolekülen aus flüssigen Medien. WO 2004/103552/ beschreibt ein ähnliches Verfahren.

Bei der Herstellung flüssiger Nahrungsmittel, wie Wein, Bier oder klaren Fruchtsäften, werden zur Stabilisierung dieser Getränke und zur Verbesserung der optischen Erscheinung Proteine, welche noch in den Getränken enthalten sind, entfernt. Die Proteine wirken als Störstoffe, die bei einer längeren Lagerung bspw. ausfallen können oder durch Zersetzung zu einer Beeinträchtigung der Qualität des Lebensmittels führen können. Es müssen bei diesem Reinigungsprozess relativ große Flüssigkeitsvolumen verarbeitet werden, um geringe Mengen an Proteinen abzutrennen. Ein ähnliches Problem tritt bei der Herstellung von bspw. Enzymen in Bioreaktoren auf, wobei das Protein als wertvoller Stoff aus einer großen Menge an flüssigem Medium entfernt werden muss.

Natriumbentonit besitzt die Eigenschaft, in Wasser stark zu quellen. Werden wenige Gramm Natriumbentonit in eine wässrige Lösung eingebracht, führt dies zu einem starken Anstieg der Viskosität, was die Handhabung der flüssigen Medien stark erschwert. Ferner lässt sich Natriumbentonit nur mit hohem Aufwand als Filtermedium oder als Packung in einer Chromatographiesäule verwenden, da durch die starke Quellung das Volumen der Filterpackung bzw. der Säulenpackung stark zunimmt und der Filter bzw. die Säule dadurch verstopft wird. Für eine technische Anwendung besteht daher bisher nur die Möglichkeit, den Natriumbentonit jeweils batchweise zum zu reinigenden flüssigen Medium zu geben und nach der Adsorption der Proteine den Bentonit wieder abzufiltrieren bzw. das gereinigte flüssige Medium durch Abpressen vom Bentonit abzutrennen.

Über die Verwendung von Schichtsilikaten zur Abtrennung von Enzymen aus dem filtrierten Saft homogenisierter Zuckerrüben berichten C. Buttersack, K. Nowikow, A. Schaper und K. Buchholz (Zuckerind. 119 (1994) Nr. 4, S. 284-291). Durch Adsorption an Natriumbentonit und anschließende Desorption mit einem pH-Puffer konnten auf einfache Weise Enzyme in reiner Form abgetrennt werden. Die Arbeiten zeigen die gute Eignung von Schichtmineralien zur Abtrennung bzw. Anreicherung von Proteinen. Die Handhabung der Schichtmineralien ist jedoch kompliziert, da diese in Wasser zu einer starken Erhöhung der Viskosität der Aufschlämmung führen und die Schichtmineralien in sehr kleine Partikel zerfallen. Es ist daher sehr schwierig, die Anreicherung von Proteinen in der Weise durchzuführen, dass das Schichtmineral in eine Säule gepackt wird, über welche dann die das Protein enthaltende Flüssigkeit geleitet wird. Ohne Hilfsmaßnahmen verstopft die Säule relativ rasch, da das Schichtmineral stark quillt. Um dennoch eine Abtrennung der Enzyme in einer Säule zu ermöglichen, schlagen die Autoren vor, den Natriumbentonit in ein Calciumalginatgel einzugießen. Das hydrierte Homogenat wird bei pH = 5,0 über die Säule gepumpt, die mit Perlen des immobilisierten Bentonits gefüllt ist. Nachdem die nicht adsorbierte Fraktion aus der Säule ausgetreten ist, wird mit einer auf pH = 8 eingestellten Pufferlösung das Enzym eluiert. Für die großtechnische Abtrennung von Proteinen ist die Verwendung von Natriumbentonit, welcher wie von den Autoren beschrieben, in einem Calciumalginat fixiert ist, wegen der hohen anfallenden Kosten wenig geeignet.

In der DE 1 160 812 wird ein Verfahren zur Erhöhung der Eiweißstabilität von Bier beschrieben. Dazu wird das Bier mit einem feinpulvrigen, weitgehend dehydratisierten und damit lagerfähigen Kieselsäurexerogel behandelt, das eine innere Oberfläche von 200 bis 400 $m^2$/g, ein Porenvolumen von mehr als 0,6 ml/g und einen Porendurchmesser von mehr als 60 Å aufweist. Um die Anforderungen des Reinheitsgebotes zu erfüllen, wird das Kieselgel sorgfältig gewaschen, sodass der Anteil an wasserlöslichen Stoffen bis auf unter 1 % abgesenkt wird.

In der DE 1 717 084 A wird ein Verfahren zur Erhöhung der Eiweißstabilität und der Verbesserung der biologischen Haltbarkeit von Bier beschrieben. Dazu wird das Bier mit feinstgemahlenem weit- bis mittelporigem Kieselgel behandelt, das nach ausreichender Einwirkzeit wieder vom Bier abgetrennt wird. Das feinstgemahlene weit- bis mittelporige Kieselgel soll zu 75 bis 90 Gew.-% eine Teilchengröße von unter 44 μm aufweisen.

In der DE 1 717 085 B wird ein Adsorptionsmittel für die Behandlung gegorener Getränke, insbesondere Bier, beschrieben. Das Adsorptionsmittel beruht auf säureaktivierten silikatischen Schichtmineralien mit quellbarem Gitter. Bevorzugt werden Montmorin-Mineralien, wie z.B. Montmorillonit und Hektorit eingesetzt, die im gequollenen Zustand einer Säureaktivierung unterworfen wurden und deren Säureaufschluss unter Erhaltung der gequollenen Gitterstruktur gewaschen und abgepresst worden ist. Das Adsorptionsmittel besitzt ein Porenvolumen von 0,3 bis 0,8, vorzugsweise 0,4 bis 0,7 ml/g, festgestellt an einer Probe, die für die Analyse bei 130°C getrocknet wurde. Die Herstellung des Adsorptionsmittels erfolgt durch Extrahieren der silikatischen Schichtmineralien mit siedender Mineralsäure, wobei Menge und Konzentration der Mineralsäure, Erhitzungsdauer, Temperatur und Druck so eingestellt werden, dass das gewünschte Porenvolumen eingestellt wird.

In der DE-OS-15 17 888 wird ein Adsorptionsmittel für die Behandlung gegorener Getränke, insbesondere Bier, beschrieben. Wie bei der DE 1 717 085 B wird ein mit Säure aufgeschlossenes silikatisches Schichtmineral verwendet, welches ein Porenvolumen von 0,3 bis 0,8, vorzugsweise 0,4 bis 0,7 ml/g aufweist, festgestellt an einer Probe, die für die Analyse bei 130°C getrocknet wurde. Das aufgeschlossene Schichtmineral wird vor der Anwendung bis zu einem Feststoffgehalt von bis zu 80 Gew.-% partiell getrocknet. Gemäß einer bevorzugten Ausführungsform des Adsorptionsmittels wird diesem eine Wasserglaslösung zugegeben, sodass es einen Anteil an Kieselsäurehydrat besitzt.

Durch den Zusatz von Wasserglas modifiziertes Adsorptionsmittel lässt sich leichter und feiner zerkleinern und als Folge in Flüssigkeiten besonders leicht verteilen. Dadurch wirkt es besonders fördernd auf die Klärung von beispielsweise Bier.

In der DE 1 642 767 B wird die Verwendung eines Adsorptionsmittel für die Klärung gegorener Getränke, wie Bier oder Wein, beschrieben, wobei das Adsorptionsmittel aus sauer aktivierten Montmorin-Mineralien hergestellt wird, die nach einem Säureaufschluss mit wässrigen oder sauren Lösungen von polymeren Phosphaten oder Polyphosphorsäuren behandelt worden sind.

In der DE 102 37 518 A1 wird die Verwendung von Schichtdoppelhydroxiden zur An- bzw. Abreicherung von Biomolekülen aus flüssigen oder fluiden Medien beschrieben. Als Sorptionsmittel wird ein Schichtdoppelhydroxid verwendet, das ausgewählt ist aus der Gruppe der natürlichen und synthetischen Hydrotalcite sowie der hydrotalcitähnlichen Verbindungen. Das Schichtdoppelhydroxid wird vor der Verwendung calciniert und weist eine mittlere Teilchengröße $D_{50}$ von mehr als 1 μm auf. Die Schichtdoppelhydroxide wirken als Anionenaustauscher. Schichtdoppelhydroxide sind kationisch geladen, weshalb sie nicht als Kationenaustauscher wirken können.

In der EP 0 566 260 B1 wird ein Verfahren zur Behandlung von Flüssigkeiten unter Zuhilfenahme von Hydrotalciten beschrieben. Das als Sorptionsmittel verwendete Hydrotalcit wirkt als Anionenaustauscher.

In der DE 41 40 746 A1 wird ein Verfahren zur Sorption wenig polarer und unpolarer organischer Substanzen aus einem polaren Medium beschrieben. Als Sorptionsmittel wird eine mit anionischen Mitteln hydrophobierte kationische Schichtverbindung vom Typ des Hydrotalcits verwendet. Der Hydrotalcit wirkt als Anionenaustauscher.

In der US 5,256,300 wird ein Verfahren zur Entfernung von Organotoxinen aus Wasser beschrieben, wobei als Sorptionsmittel eine hydrotalcitartige Verbindung verwendet wird. Mit dem als Anionenaustauscher wirkenden Sorptionsmittel können beispielsweise Pestizide, Herbizide, Insektizide sowie organische Halogenverbindungen entfernt werden.

In der US 4,458,030 wird ein Adsorbens beschrieben, welches eine Hydrotalcitverbindung sowie Aktivkohle umfasst. Die Hydrotalcitverbindung wirkt wiederum als Anionenaustauscher.

In der WO 01/12570 A1 wird ein Verfahren zur Herstellung kristalliner anionischer tonhaltiger Formkörper beschrieben, welche beispielsweise dazu verwendet werden können, organische oder anorganische Verunreinigungen aus Wasser zu entfernen. Zur Herstellung der Formkörper wird zunächst eine Vormischung aus einer Aluminiumquelle und einer Magnesiumquelle hergestellt. Diese Vormischung wird dann zu einem Formkörper geformt. Die Formkörper können dann thermisch behandelt und gealtert werden, wobei die kristallinen anionischen tonhaltigen Formkörper erhalten werden. Durch die Umsetzung der Aluminium- und Magnesiumquelle erst nach der Formgebung wird ein Formkörper erzeugt, welcher eine kontinuierliche Tonphase aufweist und daher sehr stabil ist.

Schichtmineralien zeigen potentiell eine hohe Bindekapazität für Eiweiß und können relativ kostengünstig zur Verfügung gestellt werden. Sie stellen daher ein sehr attraktives Mittel zur Schönung von beispielsweise Getränken, wie Wein oder Bier dar. Allerdings stehen einer Anwendung die oben geschilderten Probleme im Wege. Die Schönung von Getränken mit Hilfe von Schichtmineralien lässt sich nur unter großem Aufwand mit Hilfe von chromatographischen Säulen durchführen. Wird das Schichtmineral direkt dem zu schönenden Getränk zugegeben, lässt es sich nur schwierig wieder durch Filtration abtrennen, da durch die Feinteiligkeit der Partikel ein Filter relativ rasch verstopft.

Der Erfindung lag die Aufgabe zugrunde, ein Verfahren bereitzustellen, mit welchem auch in großtechnischem Maßstab eine Abtrennung von Störstoffen, insbesondere Proteinen, aus flüssigen Medien gelingt, wobei das Verfahren nach Möglichkeit kontinuierlich durchführbar sein soll.

Die Aufgabe wird mit einem Verfahren mit den Merkmalen des Patentanspruchs 1 gelöst. Vorteilhafte Weiterbildungen des erfindungsgemäßen Verfahrens sind Gegenstand der abhängigen Ansprüche.

Bei dem erfindungsgemäßen Verfahren zur Abtrennung von Biomolekülen aus flüssigen Medien wird

- ein Biomoleküle enthaltendes flüssiges Medium bereitgestellt,

- das flüssige Medium mit einem Tonmaterial behandelt, welches

  - eine spezifische Oberfläche von mehr als 150 $m^2$/g,

  - ein Porenvolumen von mehr als 0,35 ml/g, vorzugsweise mehr als 0,5 ml/g;.

  - eine Ionenumtauschfähigkeit von mehr als 40 meq/100 g, und

  - ein Sedimentvolumen bei Stehen lassen in Wasser bei Raumtemperatur für 1 Stunde von weniger als 15 ml/2 g aufweist, und

- das gereinigte flüssige Medium von dem Tonmaterial abgetrennt.

Überraschenderweise wurde gefunden, dass das oben beschriebene Tonmaterial Biomoleküle, insbesondere Proteine, in Mengen binden kann, welche für eine technische Anwendung interessant sind. Als weiteren Vorteil weist das im erfindungsgemäßen Verfahren verwendete Tonmaterial ein geringes Sedimentvolumen von weniger als 15 ml/ 2 g, vorzugsweise weniger als 10 ml/2 g auf, d.h. es quillt in Wasser nur unwesentlich. Wegen seiner geringen Quellung ist bei der Zugabe des Tonmaterials zum zu reinigenden flüssigen Medium keine wesentliche Zunahme der Viskosität des zu reinigenden Mediums zu beobachten. Auch die Abtrennung des eingesetzten Tonmaterials von dem gereinigten flüssigen Medium ist ohne Weiteres durch Filtration möglich. Wegen des geringen Quellvermögens bewirkt das Tonmaterial keine Verstopfung des Filters. Dadurch ist beim Abfiltrieren des gereinigten flüssigen Mediums nur ein vergleichsweise niedriger Druck erforderlich.

Als flüssiges Medium kann an sich jede Flüssigkeit verwendet werden, welche aus einer biologischen Quelle stammt bzw. organische Verbindungen enthält. Dies können bspw. die bereits weiter oben beschriebenen Getränke sein. Das Verfahren lässt sich jedoch auch zur Aufarbeitung von Ansätzen aus Bioreaktoren nutzen, um die interessierenden biologisch wirksamen Stoffe aus großen Flüssigkeitsvolumina abzutrennen und aufzukonzentrieren. Eine andere Einsatzmöglichkeit sind bspw. die Abtrennung von Proteinen aus Körperflüssigkeiten, wie Harn, oder anderen biologischen Proben. Ggf. müssen die Proben in üblicher Weise vorbereitet werden, indem bspw. eine Grobfiltration durchgeführt wird oder die Probe gepuffert wird. Derartige Probenvorbereitungen sind dem Fachmann bekannt.

Als flüssiges Medium wird vorzugsweise eine wässriges Medium gewählt. Das wässrige Medium enthält vorzugsweise im Wesentlichen Wasser als Lösungsmittel. Der Wasseranteil wird bevorzugt größer als 80 Gew.-%, insbesondere größer als 90 Gew.-% und besonders bevorzugt größer als 95 Gew.-% gewählt, bezogen auf das Biomoleküle enthaltende flüssige Medium. Neben Wasser können geringe Mengen anderer Flüssigkeiten im flüssigen Medium enthalten sein, insbesondere Alkohole, wie Ethanol. Der Anteil dieser anderen Flüssigkeiten wird bevorzugt kleiner als 15 Gew.-%, insbesondere bevorzugt kleiner als 12 Gew.-% gewählt.

Das im erfindungsgemäßen Verfahren verwendete Tonmaterial wirkt vermutlich als Kationenaustauscher, wobei Alkali- oder Erdalkaliionen aus dem Tonmaterial freigesetzt werden und gegen Biomoleküle ausgetauscht werden. Unter Biomolekülen werden allgemein alle Moleküle verstanden, welche aus einer biologischen Quelle stammen. Die biologische Quelle kann z.B. eine Mikrobe sein, eine Pflanze oder auch ein tierischer Organismus. Die Biomoleküle können an sich eine beliebige Struktur aufweisen. Vorzugsweise weisen die Biomoleküle geladene Gruppen auf, so dass die Biomoleküle im Austausch gegen Alkali- oder Erdalkaliionen an das Tonmaterial gebunden werden können. Beispielhafte Biomoleküle sind Zucker, welche saure oder basische Funktionen aufweisen, Moleküle, welche als Bausteine Nukleotide bzw. Nucleoside enthalten, wie DNA oder RNA, oder Fettsäuren. Besonders eignet sich das erfindungsgemäße Verfahren für die Abtrennung von Proteinen.

Das im erfindungsgemäßen Verfahren verwendete Tonmaterial weist ein Porenvolumen von mehr als 0,35 ml/g, vorzugsweise mehr als 0,5 ml/g (bestimmt nach der BJH-Methode (kumulatives Porenvolumen für Poren mit einem Durchmesser im Bereich von 1,7 bis 300 nm)), eine spezifische Oberfläche (BET-Fläche) von mehr als 150 m$^2$/g, vorzugsweise mehr als 180 m$^2$/g, insbesondere bevorzugt mehr als 200 m$^2$/g und eine Ionenumtauschfähigkeit von mehr als 40 meq/100 g auf. Ferner zeichnet sich das Tonmaterial durch ein sehr geringes Quellvermögen auf. Geeignete analytische Methoden zur Bestimmung des Porenvolumens, der spezifischen Oberfläche, der Ionenumtauschfähigkeit sowie des Quellvermögens sind nachstehend bei den Beispielen angegeben.

Besonders bevorzugt werden Tonmaterialien verwendet, deren Ionenumtauschfähigkeit über 50 meq/100 g, vorzugsweise im Bereich von 55 bis 75 meq/100 g liegt. Vorzugsweise weist das Tonmaterial eine spezifische Oberfläche (BET) im Bereich von 150 bis 280 m$^2$/g, insbesondere bevorzugt im Bereich von 170 bis 260 m$^2$/g auf. Das Porenvolumen des verwendeten Tonmaterials liegt vorzugsweise im Bereich von mehr als 0,35 ml/g, bevorzugt mehr als 0,5 ml/g, insbesondere bevorzugt im Bereich von 0,7 bis 1,0 ml/g, und ganz besonders bevorzugt im Bereich von 0,80 bis 1,0 ml/g.

Wie bereits weiter oben erläutert, quillt das im erfindungsgemäßen Verfahren verwendete Tonmaterial nur in sehr geringem Ausmaß, wodurch keine Probleme mit der Erhöhung der Viskosität einer wässrigen Suspension auftreten, wie dies beispielsweise bei Natriumbentonit beobachtet wird. Vorzugsweise beträgt das Sedimentvolumen, nachdem das Tonmaterial bei Raumtemperatur für drei Tage in Wasser stehen gelassen wurde, weniger als 15 ml/2g, vorzugsweise weniger als 10 ml/2g. Unter Raumtemperatur wird eine Temperatur von etwa 15 bis 25°C, insbesondere etwa 20°C verstanden.

Als Tonmaterialien werden bevorzugt natürlich vorkommende naturaktive oder nicht naturaktive Tonmaterialien verwendet, die vorzugsweise noch keiner chemischen Modifikation unterworfen wurden, insbesondere nicht mit starken Säuren dealuminiert wurden. Die Tonmaterialien können ggf. getrocknet und auf eine geeignete Korngröße gemahlen werden. Die Korngröße ergibt sich für den Fachmann aus der beabsichtigten Anwendung.

Neben den natürlich vorkommenden Tonmaterialien können auch synthetisch hergestellte Tonmaterialien verwendet werden, welche die oben angegebenen Eigenschaften aufweisen. Solche Tonmaterialien können beispielsweise aus Wasserglas und einem geeigneten Schichtsilikat, wie Bentonit, hergestellt werden. Bevorzugt werden jedoch natürlich vorkommende Tonmaterialien verwendet.

Besonders bevorzugt werden Tonmaterialien verwendet, deren Aluminiumgehalt, bezogen auf das wasserfreien Tonmaterial und berechnet als $Al_2O_3$, weniger als 11 Gew.-% beträgt.

Besonders bevorzugt werden Tonmaterialien verwendet, welche nur eine geringe Kristallinität aufweisen, also an sich nicht der Klasse der Schichtsilikate zugeordnet werden. Die geringe Kristallinität lässt sich bspw. durch Röntgendiffraktometrie feststellen. Die besonders bevorzugten Tonmaterialien sind weitgehend röntgenamorph, d.h. sie zeigen im Röntgendiffraktogramm keine scharfen Peaks. Sie gehören daher nicht der Klasse der reinen Attapulgite oder Smektite an.

Ohne an diese Theorie gebunden sein zu wollen, nehmen die Erfinder an, dass das im erfindungsgemäßen Verfahren verwendete Tonmaterial ein Gerüst umfasst, das aus Kieselgel gebildet ist. In dieses relativ starre Gerüst ist ein Schichtsilikat eingelagert. Durch die Verankerung im Gerüst des Kieselgels kann das Schichtmineral stark quellen, ohne dass dadurch das Mineral in seiner Gesamtheit stark in seinem Volumen zunimmt. Das aufgequollene Schichtsilikat steht dann in hohem Ausmaß für eine Adsorption von Proteinen zu Verfügung. Das Kieselgel stellt die Hauptphase dar und ist verantwortlich für das Kationenaustauschvermögen.

Das im erfindungsgemäßen Verfahren verwendete Tonmaterial weist in einer bevorzugten Ausführungsform eine besondere Porenradienverteilung auf. Der wesentliche Anteil des Porenvolumens des Tonmaterials wird dabei von Poren gebildet, welche einen Porendurchmesser von mindestens 14 nm aufweisen.

Besonders bevorzugt sind mindestens 40 % des Gesamtporenvolumens (bestimmt gemäß der BJH-Methode, vgl. unten) von Poren gebildet, die einen Durchmesser von mehr als 14 nm aufweisen. Bevorzugt werden mehr als 50 %, und insbesondere bevorzugt mehr als 60 % des Gesamtporenvolumens von Poren gebildet, die einen Durchmesser von mehr als 14 nm aufweisen. Die Porenradienverteilung bzw. das Gesamtporenvolumen wird durch Stickstoffporosimetrie (DIN 66131) und Auswertung der Adsorptionsisotherme nach der BJH-Methode (vgl. unten) bestimmt. Dies unterscheidet die im erfindungsgemäßen Verfahren verwendeten Tonmaterialien von Tonmaterialien, die durch Extraktion natürlicher Tonmaterialien mit starken Säuren, wie Salzsäure, Schwefelsäure oder Phosphorsäure, bevorzugt durch Extraktion bei erhöhter Temperatur, wie Siedehitze, erhalten werden. Bei diesen auch als hochaktive Bleicherden bezeichneten Tonmaterialien wird das Gesamtporenvolumen im Wesentlichen von sehr kleinen Poren gebildet, die vorzugsweise einen Porenradius von weniger als 14 nm aufweisen.

Nach einer weiteren Ausführungsform kann das im erfindungsgemäßen Verfahren verwendete Tonmaterial durch eine Oberflächenbehandlung mit Säure aktiviert sein. Die Aktivierung des Tonmaterials erfolgt dabei durch eine sogenannte "In situ-Aktivierung". Dabei wird das Tonmaterial mit einer Säure belegt, wobei jedoch im Wesentlichen keine Dealuminierung erfolgt. Eine solche Aktivierung von Tonmaterialien ist bspw. aus der Bleicherdeproduktion bekannt. Eine solche Aktivierung mit Säure kann beispielsweise durch Besprühen des Tonmaterials mit verdünnter Säure, bspw. Schwefelsäure oder Phosphorsäure, oder durch Vermahlen mit einer festen organischen Säure, bspw. Zitronensäure, erfolgen.

Für die Aktivierung mit einer wässrigen Lösung einer Säure bzw. einer konzentrierten Säure kann die Säure an sich beliebig gewählt werden. Es können sowohl Mineralsäuren, als auch organische Säuren oder Gemische der vorstehenden Säuren verwendet werden. Es können übliche Mineralsäuren verwendet werden, wie Salzsäure, Phosphorsäure oder Schwefelsäure, wobei Schwefelsäure bevorzugt ist. Es können konzentrierte oder verdünnte Säuren bzw. Säurelösungen verwendet werden. Als organische Säuren können Lösungen von z.B. Zitronensäure oder Oxalsäure verwendet werden. Bevorzugt ist Zitronensäure.

Im Prinzip kann für die Aktivierung mit Säure jedes dem Fachmann geläufige Verfahren verwendet werden, einschließlich der in der WO 99/02256, der US 5,008,226 und der US 5,869,415 beschriebenen Verfahren, die insoweit ausdrücklich durch Bezugnahme in die Beschreibung aufgenommen werden.

Wird die Aktivierung des Tonmaterials durch eine Behandlung mit Säure durchgeführt, wird das Tonmaterial mit einer anorganischen oder organischen Säure in Kontakt gebracht. Beispielsweise kann der Rohton dazu mit einer festen Säure, beispielsweise einer festen organischen Säure, wie Zitronensäure, vermischt werden. Bevorzugt wird dazu das Tonmaterial zusammen mit der festen Säure vermahlen, so dass ein inniger Kontakt zwischen Tonmaterial und Säure erreicht wird. Die Menge an zugegebener Säure wird bevorzugt geringer als 20 Gew.-%, insbesondere bevorzugt geringer als 10 Gew.-% und insbesondere bevorzugt geringer als 5 Gew.-% gewählt, bezogen auf das Gewicht des wasserfreien Tonmaterials. Vorzugsweise beträgt der Anteil der zugegebenen Säure zumindest 1 Gew.-%.

Bei einer anderen Ausführungsform des erfindungsgemäßen Verfahrens wird die Aktivierung des Tonmaterials in wässriger Phase durchgeführt. Dazu wird die Säure als wässrige Lösung mit dem Tonmaterial in Kontakt gebracht. Es kann dabei so vorgegangen werden, dass zunächst das Tonmaterial, welches vorzugsweise in Form eines Pulvers bereitgestellt wird, in Wasser aufgeschlämmt wird. Anschließend wird die Säure in vorzugsweise konzentrierter Form zur Suspension gegeben. Das Tonmaterial kann jedoch auch direkt in einer wässrigen Lösung der Säure aufgeschlämmt werden, oder die wässrige Lösung der Säure auf das Tonmaterial aufgegeben werden. Nach einer vorteilhaften Ausführungsform kann die wässrige Säurelösung beispielsweise auf ein vorzugsweise gebrochenes oder pulverförmiges Tonmaterial aufgesprüht werden, wobei die Wassermenge bevorzugt möglichst gering gewählt wird und z.B. eine konzentrierte Säure bzw. Säurelösung eingesetzt wird. Die Säuremenge kann vorzugsweise zwischen 1 und 10 Gew.-%, besonders bevorzugt zwischen 2 und 6 Gew.-% einer, vorzugsweise starken, Säure, insbesondere einer Mineralsäure, wie Schwefelsäure, bezogen auf das wasserfreie Tonmaterial (atro), gewählt werden. Soweit erforderlich, kann überschüssiges Wasser abgedampft werden und das aktivierte Tonmaterial dann bis zur gewünschten Feinheit gemahlen werden. Nach Aufgabe der wässrigen Lösung der Säure kann auch, soweit erforderlich, bis zum Erreichen des gewünschten Feuchtigkeitsgehalts getrocknet werden.

Dem Fachmann sind an sich derartige Aktivierungsverfahren bekannt. Bevorzugt wird das Tonmaterial jedoch ohne vorherige Aktivierung eingesetzt.

Es ist nicht erforderlich, dass die nach der Aktivierung auf der Oberfläche des Tonmaterials verbleibende überschüssige Säure bzw. die bei der Aktivierung entstehenden Salze ausgewaschen werden. Nach der Aufgabe der Säure kann das behandelte Tonmaterial unmittelbar getrocknet und dann auf die gewünschte Korngröße vermahlen werden. Beim Vermahlen werden übliche Korngrößen eingestellt. Bei einer typischen Feinheit liegt der Trockensiebrückstand auf einem Sieb mit der Maschenweite von 36 $\mu$m im Bereich von 20 bis 40 Gew.-%. Der Trockensiebrückstand auf einem Sieb mit einer Maschenweite von 25 $\mu$m liegt im Bereich von 50 bis 65 Gew.-%. Diese Feinheiten werden vorzugsweise auch bei Verwendung des nicht säureaktivierten Tonmaterials eingestellt.

Wie bereits erläutert, zeichnet sich das im erfindungsgemäßen Verfahren verwendete Tonmaterial durch ein sehr geringes Quellvermögen aus. Aus dem Tonmaterial kann daher eine Filterpackung hergestellt werden, durch welche das flüssige Medium geleitet wird, aus welchem die Biomoleküle entfernt werden sollen. Die Filterpackung kann dabei an sich jede beliebige Form annehmen. Geeignet sind bspw. Filterpatronen, welche mit dem Tonmaterial gefüllt sind. Nachdem die Aufnahmekapazität der Filterpatrone erschöpft ist, kann diese gegen eine neue Patrone ersetzt werden. Die mit den Biomolekülen beladene Filterpatrone kann entweder verworfen werden oder einer Aufarbeitung zugeführt werden, in welcher die auf dem Tonmaterial absorbierten Biomoleküle wiedergewonnen werden.

Gemäß einer bevorzugten Ausführungsform wird die Filterpackung durch eine Anschwemmfiltration hergestellt. Das Tonmaterial wird dabei vorzugsweise mit einem Filterhilfsmittel, wie Kieselgur oder Perlit gemischt. Ferner kann das Tonmaterial auch noch mit einem weiteren Adsorbens, wie Kieselgel oder sauer aktiviertem Bentonit gemischt sein. Bei der Filtration bildet das Tonmaterial zusammen mit dem Filterhilfsmittel und ggf. den weiteren Adsorbentien einen Filterkuchen aus, der das filtrationswirksame Medium für nachfolgende Trübstoff- und Filterhilfsmittelteilchen bildet.

Bevorzugt wird vor der direkten Filtration eine Filterhilfsmittelschicht als Primärschicht auf ein Filtermittel aufgebracht. Als Filtermittel kann beispielsweise ein Sieb mit einer geeignet geringen Maschenweite verwendet werden. Als Filterhilfsmittel zur Ausbildung einer Primärschicht kann beispielsweise Kieselgur verwendet werden. Dieser Prozess wird auch als Voranschwemmung bezeichnet. In der anschließenden Filtrationsphase wird der Suspension weiteres Filterhilfsmittel zugesetzt, das erfindungsgemäß das oben beschriebene Tonmaterial umfasst. Diese Filterhilfsmittelteilchen bilden gemeinsam mit den Trübstoffteilchen einen Filterkuchen aus, der als Sekundärschicht bezeichnet wird.

Durch die Verwendung des Tonmaterials als Filtermaterial kann das erfindungsgemäße Verfahren auch in kontinuierlicher Form durchgeführt werden, was bspw. insbesondere für die Klärung von Getränken von Vorteil ist.

Gemäß einer weiteren Ausführungsform des erfindungsgemäßen Verfahrens wird dieses in Form einer Chromatographie durchgeführt.

Dabei wird das Tonmaterial in eine Chromatographiesäule gepackt und das flüssige Medium, aus welchem die Biomoleküle abgetrennt werden sollen, auf die Säule gegeben. Die Säule kann dann von einem Elutionsmittel durchströmt werden, wobei durch Adsorptionseffekte eine Auftrennung der Biomoleküle in unterschiedliche Fraktionen möglich ist. Dies ist insbesondere interessant für die Abtrennung von Proteinen, welche in Bioreaktoren erzeugt wurden. Auch hier zeigen sich die Vorteile des im erfindungsgemäßen Verfahren verwendeten Tonmaterials, das sich durch ein sehr geringes Quellvermögen auszeichnet.

Die Eluierung der Biomoleküle kann dabei nach verschiedenen Verfahren erfolgen. Bspw. kann ein Salzgradient über die Säule geleitet werden, so dass die Biomoleküle bei unterschiedlicher Salzkonzentration eluiert werden. Biomoleküle, wie beispielsweise Proteine, werden unter anderem durch elektrische Wechselwirkungen und/oder van-der-Waals-Kräfte an das im erfindungsgemäßen Verfahren verwendete Tonmaterial gebunden. Bei der Eluierung mit einem Puffer mit hoher Salzkonzentration werden die elektrostatischen Wechselwirkungen zwischen dem Tonmaterial, welches als Kationenaustauscher wirkt, und dem Protein herabgesetzt. Das Protein kann daher vom Tonmaterial abgelöst und eluiert werden.

Nach einer weiteren Ausführungsform erfolgt die Eluierung der gebundenen Biomoleküle durch eine Änderung des pH-Wertes. Dabei weist das Elutionsmittel einen zum flüssigen Medium unterschiedlichen pH-Wert auf.

Bspw. können Proteine bei einem pH aufgegeben werden, bei welchem diese in geladener Form vorliegen. Der pH-Wert der Lösung wird dabei vorzugsweise so eingestellt, dass er unterhalb des isoelektrischen Punktes des Proteins liegt, das Protein also eine positive Gesamtladung aufweist. Durch die Kationenaustauscherwirkung des Tonmaterials werden die Proteine gebunden. Das im erfindungsgemäßen Verfahren verwendete Tonmaterial wirkt also als Kationenaustauschermaterial. Wird nun der pH soweit angehoben, dass der isoelektrische Punkt des Proteins erreicht oder überschritten wird, weist das Protein in den meisten Fällen eine negative Gesamtladung auf. Das Protein wird daher wieder vom Tonmaterial bzw. der Filterschicht abgelöst und kann eluiert werden.

Ggf. kann dem Elutionsmittel Glycerin beigegeben sein. Glycerin weist eine sehr hohe Affinität zum Tonmaterial auf und kann daher die Ablösung des Proteins bzw. der organischen Verbindungen vom Tonmaterial erleichtern.

Das flüssige Medium umfasst bevorzugt Wasser als Lösungsmittel.

Das im erfindungsgemäßen Verfahren verwendete Tonmaterial kann allein oder auch in Kombination mit einem weiteren Adsorptionsmittel verwendet werden. Das weitere Adsorptionsmaterial ist vorzugsweise ausgewählt aus der Gruppe, die gebildet ist aus Kieselgel, Cellulose und Polyvinylpyrrolidon.

Das Tonmaterial und das weitere Adsorptionsmittel liegen bevorzugt in einem Verhältnis zwischen 1 :10 und 10 : 1 vor. In Abhängigkeit von der beabsichtigten Anwendung können jedoch auch Verhältnisse außerhalb des angegebenen Bereichs zur Anwendung gelangen.

Das im erfindungsgemäßen Verfahren verwendete Tonmaterial weist eine sehr hohes Tragevermögen für Wasser auf, wobei es dennoch fließfähig bleibt. Bevorzugt weist das Tonmaterial einen Wassergehalt von mehr als 30 Gew.-%, insbesondere mehr als 40 Gew.-%, insbesondere bevorzugt mehr als 50 Gew.-% auf.

Beispielsweise werden in der Bierfiltration Xerogele verwendet, die eine hohe spezifische Oberfläche und einen geringen Wassergehalt aufweisen. Der Wassergehalt dieser Xerogele liegt im Bereich von etwa 10 bis 15 %. Diese Xerogele werden vor allem in geschlossenen Systemen verwendet. Wegen der Staubentwicklung werden bei offenen Systemen bevorzugt Semihydrogele mit einem Wassergehalt im Bereich von etwa 30 - 40 Ges.-% oder Hydrogele mit einem Wassergehalt von mehr als 55 Gew.-% verwendet. Das im erfindungsgemäßen Verfahren verwendete Tonmaterial eignet sich daher wegen seiner hohen Wasseraufnahmekapazität besonders für die Herstellung von Mischungen mit Semihydrogelen sowie Hydrogelen, wobei die Mischung dann im erfindungsgemäßen Verfahren eingesetzt werden kann.

Zur Herstellung derartiger Mischungen kann das Tonmaterial beispielsweise in einem Intensivmischer vorgelegt und dann mit der entsprechenden Menge an Wasser beaufschlagt werden. Bei einer Beaufschlagung mit einer Wassermenge von beispielsweise 50 Gew.-% wird ein fließfähiges Pulver erhalten, welches alleine oder in Mischung mit anderen Absorptionsmitteln, insbesondere Semihydrogelen und Hydrogelen im erfindungsgemäßen Verfahren eingesetzt werden kann.

Das im erfindungsgemäßen Verfahren verwendete Tonmaterial weist vorteilhaft eine geringe Metallauslaugung auf. Bevorzugt beträgt die Schwermetallauslaugung weniger als 1,5 ppm/100g Arsen und weniger als 10 ppm/100 g Blei. Ein Verfahren zur Bestimmung der Metallauslaugung ist bei den Beispielen angegeben.

Das erfindungsgemäße Verfahren eignet sich besonders für die Filtration von Bier. Dabei wird vorzugsweise das Bier mit dem Tonmaterial behandelt, indem das Tonmaterial zu dem Bier gegeben wird, sodass eine Suspension erhalten wird. Dem Tonmaterial kann dabei ggf. Kieselgel als weiteres Adsorptionsmittel beigegeben sein. Das Tonmaterial wird anschließend vorzugsweise durch eine Anschwemm/Kuchenfiltration aus der Suspension abgetrennt.

In der Brauerei geht man dabei vorteilhaft wie folgt vor: Auf einem Metallsieb oder einer Filterschicht aus Cellulose wird zunächst eine Voranschwemmung mit Kieselgur durchgeführt. Hierzu wird Kieselgur in Wasser suspendiert und die Suspension durch das Metallsieb bzw. die Filterschicht gepumpt, sodass eine dünne Kieselgurschicht erhalten wird. Dies dient dazu, ein Durchbrechen der Mischung aus Tonmaterial und Kieselgur zu verhindern. Im Allgemeinen werden für die Filtration des Biers Siebe mit einer Maschenweite im Bereich von etwa 45 μm verwendet. In das Bier wird das Tonmaterial, ggf. in Mischung mit Kieselgur, eingerührt und die erhaltene Biersuspension durch den mit einer Voranschwemmschicht bedeckten Filter gepumpt. In der sich aus dem Tonmaterial und ggf. Kieselgur ausbildenden Schicht werden dann Trübstoffe, wie Proteine oder Protein-Polyphenolkomplexe, in adsorbierter Form zurückgehalten. Der Filtriervorgang wird auch als Kuchenfiltration bezeichnet.

Gemäß einer weiteren Ausführungsform kann das erfindungsgemäße Verfahren zur Abwasserreinigung eingesetzt werden, insbesondere zur Abtrennung von Proteinen oder Mikroorganismen. Das Abwasser kann dazu, ggf. nach einer Vorreinigung, über eine Filterpackung geleitet werden, welche aus dem im erfindungsgemäßen Verfahren verwendeten Tonmaterial besteht bzw. dieses enthält. Dies ermöglicht eine einfache und kostengünstige Verringerung der Fracht des Abwassers an organischen Verunreinigungen, insbesondere Biomolekülen. Beispielhafte Abwässer, die mit dem erfindungsgemäßen Verfahren gereinigt werden können, sind Abwässer, wie sie bei Fermentationsprozessen oder in Brauereien anfallen.

Das erfindungsgemäße Verfahren wird an Hand der folgenden Beispiele sowie unter Bezugnahme auf die beigefügten Figuren näher erläutert. Dabei zeigt:

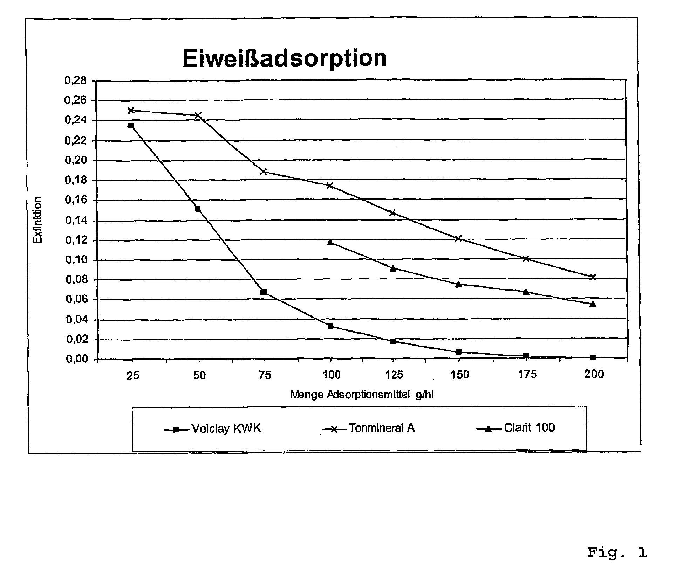

Fig. 1:    eine Grafik, in welcher die nach der Eiweißentfernung bestimmte Trübung gegen die Menge an eingesetztem Adsorptionsmittel aufgetragen ist, wobei als Adsorptionsmittel ein Tonmaterial, wie es für das erfindungsgemäße Verfahren geeignet ist, sowie ein Natriumbentonit und ein Calciumbentonit verwendet wurden;

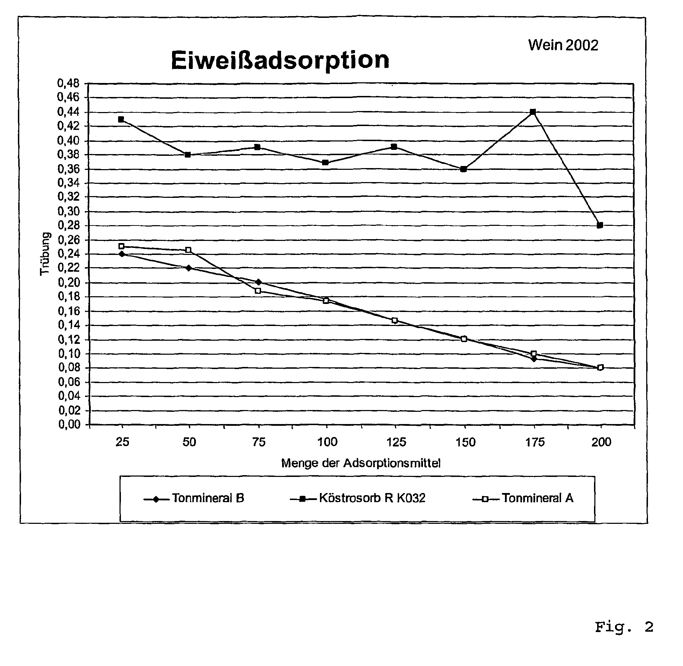

Fig. 2:    eine Grafik, in welcher die nach der Eiweißentfernung bestimmte Trübung gegen die Menge an eingesetztem Adsorptionsmittel aufgetragen ist, wobei als Adsorptionsmittel zwei Tonmaterialien, wie sie für das erfindungsgemäße Verfahren geeignet sind, sowie als Vergleich ein Kieselgel verwendet wurden.

Es wurden die folgenden Analysenmethoden verwendet.

Oberfläche/Porenvolumen:

Die Oberfläche wurde an einem vollautomatischen Stickstoffporosimeter der Firma Micromeritics, Typ ASAP 2010, gemäß DIN 66131 durchgeführt. Das Porenvolumen wurde unter Anwendung der BJH Methode ermittelt (E.P Barrett, L.G. Joyner, P.P. Haienda, J. Am. Chem. Soc. 73 (1951) 373). Porenvolumina bestimmter Porengrößenbereiche werden durch Aufsummieren inkrementeller Porenvolumina bestimmt, die aus der Auswertung der Adsorptionsisotherme nach BJH erhalten werden. Das Gesamtporenvolumen nach der BJH-Methode bezieht sich auf Poren mit einem Durchmesser von 1,7 bis 300 nm.

Wassergehalt:

Der Wassergehalt der Produkte bei 105°C wurde unter Verwendung der Methode DIN/ISO-787/2 ermittelt.

Elementaranalyse:

Diese Analyse beruht auf dem Totalaufschluss der Tonmaterialien bzw. des entsprechenden Produktes. Nach dem Auflösen der Feststoffe werden die Einzelkomponenten mit herkömmlichen spezifischen Analysenmethoden, wie z.B. ICP, analysiert und quantifiziert.

Kationenaustauschfähigkeit:

Zur Bestimmung der Kationenaustauschkapazität wurde das zu untersuchende Tonmaterial über einen Zeitraum von zwei Stunden bei 105°C getrocknet. Danach wurde das getrocknete Tonmaterial mit einem Überschuss an wässriger 2N $NH_4$Cl-Lösung eine Stunde unter Rückfluss zur Reaktion gebracht. Nach einer Standzeit von 16 Stunden bei Raumtemperatur wurde filtriert, worauf der Filterkuchen gewaschen, getrocknet und vermahlen wurde und der $NH_4$-Gehalt im Tonmaterial durch Stickstoffbestimmung (CHN-Analysator der Fa. Leco) nach den Herstellerangaben ermittelt wurde. Der Anteil und die Art der ausgetauschten Metallionen wurde im Filtrat durch ICP-Spektroskopie bestimmt.

Röntgendiffraktometrie:

Die Röntgenaufnahmen werden an einem hochauflösenden Pulverdiffraktometer der Fa. Phillips (X'-Pert-MPD (PW 3040)) erstellt, das mit einer Cu-Anode ausgerüstet war.

Bestimmung des Sedimentvolumens

Ein graduierter 100 ml Messzylinder wird mit 100 ml destilliertem Wasser gefüllt. 2 g der zu vermessenden Substanz werden langsam und portionsweise, je etwa 0,1, bis 0,2 g, mit einem Spatel auf die Oberfläche des Wassers gegeben. Nach dem Absinken einer zugegebenen Portion wird die nächste Portion zugegeben. Nachdem die 2 g Substanz zugegeben und auf den Grund des Messzylinders abgesunken sind, wird der Zylinder für eine Stunde bei Raumtemperatur stehen gelassen. Anschließend wird an der Graduierung des Messzylinders die Höhe des Sedimentvolumens in ml/2g abgelesen. Für die Bestimmung des Sedimentvolumens nach dreitägiger Lagerung in Wasser wird der Probenansatz mit Parafilm® verschlossen und für drei Tage bei Raumtemperatur erschütterungsfrei stehen gelassen. Danach wird an der Graduierung des Messzylinders das Sedimentvolumen abgelesen.

Bestimmung der Metallauslaugung

Die Metallauslaugung wird nach der Methode bestimmt, wie sie in der deutschen Weinverordnung beschrieben ist (Bekanntmachung der Neufassung der Weinordnung vom 14. Mai 2002, Bundesgesetzblatt Jahrgang 2002 Teil I Nr. 31, ausgegeben zu Bonn am 22. Mai 2002).

2,5 g des lufttrockenen Tonmaterials werden in einem 250 ml Messkolben eingewogen und der Messkolben bis zur Markierung mit 1 %-iger Weinsäurelösung aufgefüllt. Die Suspension wird unter gelegentlichem Umschwenken 24 Stunden stehen gelassen. Die überstehende klare Lösung wird durch Abdekantieren oder Zentrifugieren vom Feststoff abgetrennt. Die in der Lösung enthaltenen Metallionen werden durch Atomabsorptionsspektroskopie bestimmt und quantifiziert.

Bestimmung des Trockensiebrückstandes

Etwa 50 g des zu untersuchenden lufttrockenen Tonmaterials werden auf einem Sieb der Maschenweite 45 μm eingewogen. Das Sieb wird an einen Staubsauger angeschlossen, der über ein unter dem Siebboden kreisenden Saugschlitz alle Anteile, die feiner als das Sieb sind, durch das Sieb heraussaugt. Das Sieb wird mit einem Plastikdeckel abgedeckt und der Staubsauger eingeschaltet. Nach 5 Minuten wird der Staubsauger abgeschaltet und die Menge der auf dem Sieb verbliebenen gröberen Anteile durch Differenzwägung ermittelt.

Bestimmung des Nasssiebrückstandes

Es wird zunächst eine 5 %-ige Suspension hergestellt, indem eine entsprechende Menge des zu untersuchenden Tonmaterials bei ca. 930 UpM ca. 5 Minuten in Wasser eingerührt wird. Die Suspension wird für weitere 15 Minuten bei ca. 1865 UpM gerührt und die Suspension dann durch ein Sieb der gewünschten Maschenweite gegossen. Der Rückstand wird so lange mit Leitungswasser gewaschen, bis das Waschwasser klar abläuft. Das Sieb mit dem Rückstand wird dann für 5 Minuten in ein Ultraschallbad gesetzt, um restliche Feinanteile zu entfernen. Der verbliebene Rückstand wird kurz mit Leitungswasser gewaschen und die Ultraschallbehandlung ggf. wiederholt, bis während der Ultraschallbehandlung keine Feinstoffe mehr in das Wasser übertreten. Das Sieb wird dann bis zur Gewichtskonstanz getrocknet. Zum Auswiegen wird der auf dem Sieb verbliebene Rückstand in eine gewogene Porzellanschale überführt.

Beispiel 1

Charakterisierung des Rohtons

(a) Tonmaterial A

Ein für das erfindungsgemäße Verfahren geeignetes Tonmaterial (erhältlich bei: Süd-Chemie AG, Moosburg DE, Rohton Ref. No.: 03051, Tonsil® 419 FF) wurde hinsichtlich seiner physikalischchemischen Eigenschaften untersucht. Die hierbei erzielten Ergebnisse sind in Tabelle 1 zusammengefasst.

Tabelle 1

| Physikalisch-chemische Analyse des Tonmaterials A | | |
|---|---|---|
| Spezifische Oberfläche | ($m^2$/g) | 219 |
| Porenvolumen[1] | (ml/g) | 0,881 |
| Ionenaustauschkapazität | (meq/100g) | 52 |
| Sedimentvolumen (1h) | (ml/2g) | 5,5 |
| Sedimentvolumen (3 Tage) | (ml/2g) | 6,5 |
| **Elementaranalyse:** | | |
| $SiO_2$ | (Gew.-%) | 70,6 |
| $Fe_2O_3$ | (Gew.-%) | 2,8 |
| $Al_2O_3$ | (Gew.-%) | 9,8 |
| CaO | (Gew.-%) | 1,4 |
| MgO | (Gew.-%) | 4,1 |
| $Na_2O$ | (Gew.-%) | 0,26 |
| $K_2O$ | (Gew.-%) | 1,5 |
| $TiO_2$ | (Gew.-%) | 0,25 |
| Glühverlust (2h 1000°C) | | 7,9 |
| **Summe** | **(Gew.-%)** | **98,6** |
| [1]: kumulatives Porenvolumen nach BHJ für Poren mit einem Durchmesser zwischen 1,7 und 300 nm. | | |

(b) Tonmaterial B

Die physikalischen Eigenschaften eines weiteren Tonmaterials, das für die Durchführung des erfindungsgemäßen Verfahrens geeignet ist, sind in Tabelle 2 zusammengefasst. Das in den Beispielen eingesetzte Material wies eine mittlere Teilchengröße von 15 μm auf.

Tabelle 2

| Physikalisch-chemische Analyse des Tonmaterials B | | |
|---|---|---|
| Spezifische Oberfläche | ($m^2$/g) | 198 - 206 |
| Porenvolumen[1] | (ml/g) | 0,402 |
| Ionenaustauschkapazität | (meq/100g) | 62 |
| Sedimentvolumen (1h) | (ml/2g) | 7,0 |
| **Elementaranalyse:** | | |
| $SiO_2$ | (Gew.-%) | 35,2 |
| $Fe_2O_3$ | (Gew.-%) | 0,93 |
| $Al_2O_3$ | (Gew.-%) | 4,6 |
| CaO | (Gew.-%) | 15,4 |
| MgO | (Gew.-%) | 16,0 |
| $Na_2O$ | (Gew.-%) | 0,47 |
| $K_2O$ | (Gew.-%) | 0,66 |
| $TiO_2$ | (Gew.-%) | 0,1 |
| Glühverlust (2h 1000°C) | | 25,4 |
| **Summe** | **(Gew.-%)** | **98,76** |

Beispiel 2

Aktivierung des Tonmaterials mit Schwefelsäure

Das in Beispiel 1 charakterisierte Tonmaterial A wurde mit Wasser vermischt und anschließend mit 3 Gew.-% $H_2SO_4$ aktiviert. Hierzu wurden 100 g auf 9,3% $H_2O$ getrocknetes Pulver mit 208 g Wasser und 2,83 g $H_2SO_4$ (96%-ig) in einem Becherglas innig vermengt. Das entstandene Gemisch wurde bei 110°C auf einen Wassergehalt von 9,4% getrocknet und anschließend auf eine typische Bleicherdefeinheit vermahlen (Trockensiebrückstand auf 63 μm Sieb: 20 bis 40 Gew.-%; Trockensiebrückstand auf 25 μm Sieb: 50 bis 65 Gew.-%).

Beispiel 3

Entfernung von Restprotein aus Weißwein

Das erfindungsgemäße Verfahren wurde zur Entfernung von Eiweiß aus Weißwein eingesetzt. Zur Analyse des Gehaltes an Restprotein wurde ein Verfahren aus dem Wein- und Bodenlabor Dr. Karl-Heinz Nilles, Volkach, verwendet. Es wurden Dr. Nilles-Reagens 1 (Blindwert) und Dr. Nilles-Reagens 2 gemäß den Angaben und der Anleitung des Herstellers zur Ermittlung des Eiweiß-Adsorptionsvermögens verwendet. Die Methode beruht auf einer Fällung des Weinproteins mit Hilfe eines eiweißspezifischen Reagens mit anschließender photometrischer Trübungsmessung der farblosen Proteinfällung bei einer Wellenlänge von 623 nm.

Das in Beispiel 1 charakterisierte Tonmaterial A wurde in Leitungswasser dispergiert und die Suspension zum Weißwein gegeben. Die Mengen betrugen ca. 50 g bis 200 g Tonmaterial/HL-Wein. Nach einer Einwirkzeit von 15 Min. wurde die Probe zur Abtrennung des Tonmaterials zentrifugiert und der klare Überstand abpipettiert. Der Eiweißgehalt im klaren Überstand wurde dann in der oben beschriebenen Weise mit dem Reagens nach Dr. Nilles bestimmt.

Der Versuch wurde auch mit dem in Beispiel 1 charakterisierten Tonmaterial B wiederholt.

Für die Untersuchungen wurden die folgenden Weine verwendet: 2002er Eschendorfer Lump, Silvaner trocken, Weingut Rainer Sauer, Eschendorf.

Beispiel 4 (Vergleich)

Beispiel 3 wurde wiederholt, wobei jedoch ein Natriumbentonit mit hohem Quellvermögen (VOLCLAY KWK) und eine Getränkebentonitgranulat aus Calciumbentonit verwendet wurde (Clarit® 100, Süd-Chemie AG, München, DE).

In einem weiteren Versuch wurde ein kommerziell erhältliches Kieselgel (Köstrosorb® K032, KW Chemiewerk Bad Köstritz, DE-07586 Bad Köstritz) eingesetzt, welches typischerweise in der Bierstabilisation eingesetzt wird.

Die Ergebnisse aus den Beispielen 3 und 4 sind in den Figuren 1 und 2 dargestellt. Dabei ist jeweils die Extinktion gegen die zugegebene Menge an Tonmaterial (g/hl) angegeben. Bei niedrigen Extinktionskoeffizienten, welche erhalten werden, wenn alles Resteiweiß entfernt wurde, wird jeweils ein Plateau erreicht, welches der Geräteauflösung entspricht (etwa 0,01).

In Figur 1 ist die Eiweißadsorption für das Tonmaterial A sowie für Natriumbentonit (Volclay® KWK) und Calciumbentonit (Clarit® 100) dargestellt. Dazu wurde jeweils die ermittelte Trübung gegen die eingesetzte Menge des Adsorptionsmittels aufgetragen.

Die stärkste Proteinbindung wurde mit dem Natriumbentonit Volclay KWK erhalten. Der Bentonit Clarit® 100 zeigt eine deutlich niedrigere Adsorptionsfähigkeit für Proteine. Mit dem erfindungsgemäßen Verfahren werden ca. 50 % des Bindungsvermögens des Calciumbentonits erhalten.

Der Natriumbentonit Volclay KWK zeigt eine sehr hohe Quellfähigkeit. Beim Eintrag von 5 Gew.-% wird eine hochviskose Paste erhalten, welche sich nur schwer verarbeiten lässt. Bei Calciumbentonit Clarit® 100 können ca. 15 bis 20 Gew.-% Ton in die zu reinigende Lösung eingetragen werden, ehe durch eine Viskositätszunahme eine technische Verarbeitung erschwert wird.

Nur bei dem im erfindungsgemäßen Verfahren verwendeten Tonmaterial wird keine Quellung beobachtet. Das Verfahren lässt sich daher kontinuierlich durchführen, wobei hohe Konzentrationen an Tonmaterial verwendet werden können.

In Figur 2 ist die Eiweißadsorption für die Tonmaterialien A und B sowie für das Kieselgel (Köstrosorb® K032) dargestellt. Es wurde dazu jeweils die ermittelte Trübung gegen die eingesetzte Menge des Adsorptionsmittels aufgetragen.

Figur 2 zeigt, dass die Wirksamkeit des Tonmaterials B im Rahmen der Fehlergenauigkeit identisch mit der Wirksamkeit des Tonmaterials A war. Die beiden Tonmaterialien sind zur Entfernung von Resteiweiß aus Weiswein besser geeignet als Kieselgel.

Beispiel 5

Filtration von Bier

Für die Prüfung der Filtrierbarkeit von Bier wurde die Versuchsvorrichtung "Filtercheck®" der Firma Stabifix Brauerei-Technik KG, Gräfelfing, DE verwendet.

Die Versuchsvorrichtung "Filtercheck®" wurde nach Angaben des Herstellers gereinigt und zusammengesetzt. Anschließend wurde die Apparatur mittels Kühlbad und Umwälzpumpe auf eine Filtrationstemperatur von 0 °C temperiert.

500 ml Bier, aus welchem die Kohlensäure entfernt worden war, wurde in einer Bügelverschlussflasche auf 0 °C temperiert. Nachdem das Bier auf die Filtrationstemperatur abgekühlt worden war, wurden mittels eines Pulvertrichters 2,5 g Kieselgur (Filtercel®, John Manville, USA, Vertrieb in Deutschland über Lehrmann und Voss, Hamburg, Permeabilität 60 - 70 mDarcy) sowie 50 g des zu untersuchenden Adsorptionsmittel zugegeben. Die Suspension wird anschließend ca. 60 Sekunden mit einem Magnetrührer homogenisiert und dann erneut auf 0°C abgekühlt.

240 ml der Suspension wurden in die Versuchsvorrichtung überführt und diese anschließend geschlossen. Unter den Ablauf der Versuchsvorrichtung wurde ein Messzylinder gestellt. Der Ablauf wurde geöffnet und ein Druck von 1 bar an der Versuchsvorrichtung angelegt. Nachdem 40 ml Vorlauf abgelaufen waren, wurde der Messzylinder durch eine 180 ml Bügelverschlussflasche ersetzt und die Flasche mit filtriertem Bier gefüllt. Es wurde sowohl die Menge des filtrierten Biers bestimmt, als auch die Zeit, die für die Filtration benötigt wurde. Das spezifische Filtratvolumen wurde gemäß der folgenden Formel berechnet und ist in hl/m$^2$h angegeben.

$$F_{spez} = F \cdot V \cdot (t)^{-1/2}$$

V: Gesamtfiltratvolumen (ml)
t: Filtrationsdauer (s)
F: Faktor (0,346 m$^{-2}$)

Die Eiweißentfernung aus dem Rohbier lässt sich durch die Abnahme der Trübung verfolgen. Die Messung der Trübung wurde mit dem Bierphotometer LTP6B der Firma Dr. Lange bei einer Wellenlänge von 860 nm nach dem 90 °-Zweistrahl-Streulichtverfahren durchgeführt.

Die Trübung ist in EBC-Einheiten angegeben. Die EBC-Skala basiert auf einer Formazin-Standardsuspension, die mit einer Genauigkeit von ± 2 % hergestellt werden kann (DIN 38 404 C2). Dieses Konzentrat entspricht 100 EBC. Durch Verdünnung mit destilliertem Wasser können Eichlösungen mit beliebigen EBC-Werten hergestellt werden.

Die Filtrationsversuche wurden mit den folgenden Adsorptionsmitteln durchgeführt:

| Nr. | Adsorptionsmittel |
|---|---|
| 1 | 50 % Tonmaterial A, 50 % Kieselgel |
| 2 | 50 % hoch aufgeschlossene Bleicherde, 50 % Kieselgel |
| 3 | 50 % Tonmaterial A, 50 % Kieselgel, Tonmaterial A auf > 45 $\mu$m abgesiebt |

Bezugsquellen:

Kieselgel: Zeochem C560, Zeochem AG, CH

hoch aufgeschlossene Bleicherde: Die Bleicherde wurde gemäß DT 1517888 B2 aus einem Rohton hergestellt, der 70 % Montmorillonit enthielt. Sie wies eine BET-Oberfläche von 180 m$^2$/g sowie ein Schüttgewicht von 380 g/l auf. Das kumulative Porenvolumen von Poren mit einem Durchmesser von 1,7 - 300 nm betrug 0,43 cm$^3$/g. Der pH-Wert, bestimmt durch Zugabe von 10 g Ton auf 100 ml destilliertes Wasser, betrug 3.

Die Ergebnisse der Filtrationsversuche sind in Tabelle 3 zusammengefasst.

Ferner wurde die nicht-biologische Haltbarkeit des Bieres mit Hilfe des Forciertests ermittelt. Wird Bier auf 0 °C abgekühlt, kommt es zur Ausbildung einer Kältetrübung, d.h. ein Teil der Trübstoffe, vor allem Proteine und Harnstoffe, fällt aus. Diese Kältetrübung ist nur teilweise reversibel. Ein Ausfällen von Trübstoffen wird auch bei höheren Temperaturen beobachtet, wobei die Trübung umso schneller auftritt, je höher die Lagertemperatur ist. Kann die Trübung mit bloßem Auge wahrgenommen werden, ist die Grenze der nicht-biologischen Haltbarkeit erreicht.

Beim Forciertest wird das Bier künstlich gealtert, indem es bei 40°C gelagert und in regelmäßigen Abständen auf 0 °C gekühlt wird. Die Ergebnisse hierzu am Anfang und nach 2 Zyklen (2 Warmtage ; WT) sind in den beiden letzten Spalten der Tabelle 3 angegeben.

Tabelle 3: Filtration von Rohbier

| Nr. | Adsorptionsmittel | Filtrationsdaten | | | Forciertest 0°C/40°C Trübung in EBC bei 0°C | |
|---|---|---|---|---|---|---|
| | | $t_{220ml}$ (S) | $F_{spez}$ (hl/m$^2$s) | $EBC_{Filtrat}$ 15°C | 0 WT | 2 WT |
| 1 | Kein Adsorpt.mitt. | 201 | 5,4 | 0,6 | 0,6 | 8,9 |
| 2 | 50% Kieselgel 50% Bleicherde | 302 | 4,4 | 0,39 | 0,44 | 3,9 |
| 3 | 50% Kieselgel 50% Tonmaterial A | 378 | 3,9 | 0,37 | 0,41 | 3,4 |
| 4 | 50% Kieselgel 50% Tonmaterial A (>45 $\mu$m) | 297 | 4,4 | 0,43 | 0,47 | 3,9 |

Die Ergebnisse zeigen, dass das Tonmaterial A in einer Mischung mit Kieselgel zu einer vergleichbaren Filtrationsleistung führt, wie sie mit einer üblicherweise für die Bierfiltration eingesetzten Mischung aus hoch aufgeschlossener Bleicherde und Kieselgel erreicht wird. Weiterhin wird eine ähnliche Stabilisierungsleistung bezüglich der Entfernung von Resteiweiß gefunden. Weiter zeigt sich, dass bei einer Verwendung des Tonmaterials mit einer Korngröße von >45 $\mu$m eine höhere spezifische Filterleistung erreicht werden kann.

Beispiel 6: Metallauslaugung in Weinsäure

2,5 g des in Beispiel 1 charakterisierten Tonmaterials A (lufttrocken) werden in einem 250 ml Messkolben eingewogen und dieser bis zur Eichmarke mit 1 %-iger Weinsäurelösung aufgefüllt. Der Messkolben wird 24 Stunden bei Raumtemperatur stehen gelassen und dann der Kolbeninhalt über ein Faltenfilter filtriert. Im Filtrat werden die in Tabelle 4 angegebenen Werte mittels AAS bestimmt. Zum Vergleich sind auch die Grenzwerte nach dem deutschen Weingesetz mit aufgenommen.

Tabelle 4: Metallauslaugung in Weinsäure

|  | In Weinsäure | Grenzwert |
|---|---|---|
| As (ppm) | 0,9 | 2 |
| Pb (ppm) | 3 | 20 |
| Ca (%) | 1,20 | 0,8 |
| Fe (%) | 0,03 | 0,2 |
| Mg (%) | 0,13 | 0,5 |
| Na (%) | 0,05 | 0,5 |
| Cd (ppm) | 0,2 | - |
| Hg (ppm) | < 0,1 | - |

Die Daten zeigen eine sehr geringe Metallauslaugung des Tonmaterials. Insbesondere enthält das Tonmaterial A nur sehr geringe Mengen an auslaugbaren Schwermetallen. Der Wert für Calcium liegt zwar etwas oberhalb des Grenzwertes. Durch Zugabe anderer Adsorbentien, beispielsweise Kieselgel, kann der Wert jedoch ohne weiteres unter den Grenzwert abgesenkt werden. Weiter gelten für andere Anwendungen, wie die Bier- oder Fruchtsaftfiltration, andere Grenzwerte.

Beispiel 7: Bindung von Rinderserumalbumin

Es wurde die Bindung von Rinderserumalbumin als Modellsubstanz an ein im erfindungsgemäßen Verfahren verwendetes Tonmaterial sowie an Calciumbentonit und Kieselgel untersucht. Als Calciumbentonit wurde Clarit® 100, Süd-Chemie AG, München, DE, verwendet. Als Kieselgel wurde Zeochem C560, Zeochem AG, CH, verwendet.
Die Konzentrationsbestimmung des Rinderserumalbumins wurde mit dem Testreagenz Coomassie® Plus (Proteinreagenz nach Bradford) durchgeführt, das auf der Testmethode nach Bradford beruht (M. Bradford, Anal. Biochem. 72, 248 - 254 (1976). Das Reagenz wurde von der Firma Pierce Biotechnology Inc. P.O. Box 117, Rockford, IL 61105, USA bezogen.
Zunächst wurde mittels Lösungen, die eine bekannte Konzentration an Rinderserumalbumin aufwiesen, eine Eichkurve erstellt. Diese wurde dann dazu verwendet, die Rinderserumalbuminkonzentration der Proben zu ermitteln.
Zunächst wurde Rinderserumalbumin in einem Essigsäure/Acetatpuffer (Merck KgaA, Darmstadt, DE) gelöst, wobei eine Stammlösung mit einer Konzentration von 0,25 mg Rinderserumalbumin pro Milliliter erhalten wurde. Zur Durchführung der Adsorptionsexperimente wurde jeweils 25 mg Tonmaterial A in 50 ml der gepufferten Rinderserumalbuminlösung eine Stunde gerührt. Die Probe wurde anschließend bei 2500 UpM 10 Minuten zentrifugiert. Im Überstand wurde die Konzentration des nicht adsorbierten Rinderserumalbumins mit dem Bradford Testkit der Pierce Inc. nach den Angaben des Herstellers bestimmt. Aus der Differenz der Konzentration des Rinderserumalbumins in der Stammlösung und im Überstand wurde die gebundene Menge an Rinderserumalbumin bestimmt. Die Ergebnisse sind in Tabelle 5 zusammengefasst.

Tabelle 5: Adsorption von Rinderserumalbumin

| Adsorbens | Gebundene Menge an Rinderserumalbumin (mg/mg) |
|---|---|
| Calciumbentonit | 0,18 |
| Kieselgel | 0 |
| Tonmineral A | 0,15 |

Das Tonmaterial A eignet sich im Gegensatz zu Kieselgel zur Abtrennung von Rinderserumalbumin. Die Bindekapazität ist vergleichbar mit der eines Calciumbentonits.

Beispiel 8: Herstellung von säureaktiviertem Tonmaterial C

Aus dem in Beispiel 1 charakterisierten Tonmaterial A wurde durch Aufsprühen von 4 Gew.-% konzentrierter Schwefelsäure (bezogen auf die Trockenmasse des Tonmaterials A) ein säureaktiviertes Tonmaterial C hergestellt. Die Eigenschaften des Tonmaterials C sind in Tabelle 6 zusammengefasst. Das Tonmineral wurde anschließen noch fein vermahlen. Die Eigenschaften des Endproduktes (Tonmineral C) sind in der folgenden Tabelle 6 aufgelistet.

Tabelle 6: Eigenschaften des Tonmaterials C

| | | |
|---|---|---|
| Trockensiebrückstand (45 $\mu$m) | Gew.-% | 13,5 |
| Nasssiebrückstand (45 $\mu$m) | Gew.-% | 23,5 % |
| Schüttgewicht | g/l | 408 |
| Spezifische Oberfläche nach BET | $m^2$/g | 164 |
| Kationenaustauschkapazität | meq/100 g | 46 |
| Wassergehalt (2 h Trocknen bei 110 °C) | Gew.-% | 8,8 |
| pH-Wert (10 Gew.-% in Wasser) | | 2,3 |
| Kumulatives Porenvolumen nach BJH | ml/g | 0,653 |

Beispiel 9: Filtration von Bier

Analog Beispiel 5 wurden die Filtrationseigenschaften von Mischungen aus Kieselgel und dem in Beispiel 8 erhaltenen säureaktivierten Tonmaterial untersucht. Zu Vergleichszwecken wurde das Tonmaterial in der Testreihe erneut untersucht. Die Ergebnisse sind in Tabelle 7 zusammengefasst.

Tabelle 7 Filtration von Rohbier

| Nr. | Adsorptionsmittel | Filtrationsdaten | | | Forciertest* | |
|---|---|---|---|---|---|---|
| | | $T_{220\ ml}$ (S) | $F_{spez}$ (hl/$m^2$s) | $EBC_{Filtrat}$ 15°C | 0 WT | 3 WT |
| 1 | kein Adsorpt.mittel | 397 | 3,8 | 0,44 | 5,2 | -- |
| 2 | 50 % Kieselgel 50 % Bleicherde | 357 | 4,0 | 0,30 | 0,51 | 5,6 |
| 3 | 50 % Kieselgel 50 % Tonmaterial A | 389 | 3,9 | 0,32 | 0,71 | 6,0 |
| 4 | 50 % Kieselgel 50 % Tonmaterial C | 373 | 3,9 | 0,36 | 0,56 | 5,1 |

Die in Tabelle 7 wiedergegebenen Daten zeigen, dass die Tonmaterialien A und C in einer Mischung mit Kieselgel bezüglich des spezifischen Filtratvolumens identische Ergebnisse liefern wie eine Mischung aus hoch aufgeschlossener Bleicherde und Kieselgel. Bei dem in Beispiel 9 verwendeten Rohbier konnte mit dem säureaktivierten Tonmaterial C die höchste Stabilisierung erreicht werden, was sich durch die geringste Trübung nach drei Warmtagen zeigt.

**Patentansprüche**

1.  Verfahren zur Abtrennung von Biomolekülen aus flüssigen Medien, wobei

- ein Biomoleküle enthaltendes flüssiges Medium bereitgestellt wird,
- das flüssige Medium mit einem Tonmaterial behandelt wird, welches

- eine spezifische Oberfläche von mehr als 150 $m^2$/g,
- ein Porenvolumen von mehr als 0,35 ml/g
- eine Kationenaustauschfähigkeit von mehr als 40 meq/100 g und
- ein Sedimentvolumen in Wasser von weniger als 15 ml/2g aufweist, und

- das gereinigte flüssige Medium von dem Tonmaterial abgetrennt wird.

2. Verfahren nach Anspruch 1, wobei das Tonmaterial, bezogen auf das wasserfreie Tonmaterial (atro), einen $Al_2O_3$-Gehalt von weniger als 11 Gew.-% aufweist.

3. Verfahren nach Anspruch 1 oder 2, wobei das Tonmaterial, bezogen auf das wasserfreie Tonmaterial (atro) einen $SiO_2$-Gehalt von mehr als 65 Gew.-% aufweist.

4. Verfahren nach einem der vorhergehenden Ansprüche, wobei mindestens 40 % des Porenvolumens des Tonmaterials von Poren bereitgestellt werden, die einen Porendurchmesser von mindestens 14 nm aufweisen.

5. Verfahren nach einem der vorhergehenden Ansprüche, wobei das Sedimentvolumen des Tonmaterials nach Stehen lassen bei Raumtemperatur für drei Tage in Wasser weniger als 15 ml/2g, vorzugsweise weniger als 10 ml/2g beträgt.

6. Verfahren nach einem der vorhergehenden Ansprüche, wobei das Tonmaterial durch Oberflächenbehandlung mit Säure aktiviert wurde.

7. Verfahren nach einem der vorhergehenden Ansprüche, wobei das flüssige Medium zur Abtrennung der Biomoleküle durch eine Filterpackung geleitet wird, welche zumindest anteilig aus dem Tonmaterial gebildet ist.

8. Verfahren nach Anspruch 7, wobei die Filterpackung durch eine Anschwemmfiltration hergestellt wird.

9. Verfahren nach einem der vorhergehenden Ansprüche, wobei das flüssige Medium zur Abtrennung der Biomoleküle über eine Chromatographie-Säule geleitet wird, deren Packung im Wesentlichen aus dem Tonmaterial gebildet ist.

10. Verfahren nach einem der vorhergehenden Ansprüche, wobei nach der Abtrennung des gereinigten flüssigen Mediums die an das Tonmaterial gebundenen Biomoleküle mit einem Elutionsmittel eluiert werden.

11. Verfahren nach Anspruch 10, wobei das Elutionsmittel einen zum flüssigen Medium unterschiedlichen pH-Wert aufweist.

12. Verfahren nach einem der vorhergehenden Ansprüche, wobei das Biomolekül ein Protein ist.

13. Verfahren nach einem der vorhergehenden Ansprüche, wobei das flüssige Medium eine wässrige Lösung oder Suspension ist.

14. Verfahren nach einem der vorhergehenden Ansprüche, wobei das Tonmaterial im Gemisch mit einem weiteren Adsorptionsmaterial verwendet wird.

15. Verfahren nach Anspruch 14, wobei das weitere Adsorptionsmaterial ausgewählt ist aus der Gruppe, die gebildet ist aus Kieselgel, Cellulose und Polyvinylpyrrolidon.

16. Verfahren nach Anspruch 14 oder 15, wobei das Tonmaterial und das weitere Adsorptionsmittel in einem Verhältnis zwischen 1 :10 und 10 : 1 vorliegen.

17. Verfahren nach einem der vorhergehenden Ansprüche, wobei das Tonmaterial einen Wassergehalt von mehr als 30 Gew.-% aufweist.

18. Verfahren nach einem der vorhergehenden Ansprüche, wobei das Tonmaterial eine Schwermetallauslaugung von weniger als 1,5 ppm/100g Arsen und weniger als 10 ppm/100g Blei aufweist.

19. Verfahren nach einem der vorhergehenden Ansprüche, wobei das flüssige Medium ein Abwasser ist, welches organische oder mikrobielle Verunreinigungen enthält.

20. Verfahren nach einem der vorhergehenden Ansprüche, wobei das flüssige Medium Bier ist.

21. Verfahren nach Anspruch 18, wobei das Bier mit dem Tonmaterial behandelt wird, indem das Tonmaterial zu dem Bier gegeben wird, sodass eine Suspension erhalten wird, und das Tonmaterial anschließend durch eine Anschwemm/Kuchenfiltration aus der Suspension abgetrennt wird.

22. Verfahren nach Anspruch 19, wobei das Tonmaterial mit Kieselgel als weiterem Adsorptionsmittel gemischt ist.

**Claims**

1. Method for separating biomolecules from liquid media, wherein

   - a biomolecule-containing liquid medium is provided,
   - the liquid medium is treated with a clay material, which has

     - a specific surface area of more than 150 m$^2$/g,
     - a pore volume of more than 0.35 ml/g,
     - a cation exchange capacity or more than 40 meg/100 g and
     - a sediment volume in water of less than 15 ml/2 g, and

   - the purified liquid medium is separated from the clay material.

2. Method according to claim 1, wherein, relative to the anhydrous clay material (absolutely dry), the clay material has an $Al_2O_3$ content of less than 11 wt.-%.

3. Method according to claim 1 or 2, wherein, relative to the anhydrous clay material (absolutely dry), the clay material has an $SiO_2$ content of more than 65 wt.-%.

4. Method according to one of the previous claims, wherein at least 40 % of the pore volume of the clay material is provided by pores which have a pore diameter of at least 14 nm.

5. Method according to one of the precious claims, wherein the sediment volume of the clay material, after being left to stand at room temperature for three days in water, is less than 15 ml/2 g, preferably less than 10 ml/2 g.

6. Method according to one of the previous claims, wherein the clay material has been activated by surface treatment with acid.

7. Method according to one of the previous claims, wherein the liquid medium is passed, for the separation of the biomolecules, through a filter pack at least a proportion of which is formed by the clay material.

8. Method according to claim 7, wherein the filter pack is produced by a precoat filtration.

9. Method according to one of the previous claims, wherein the liquid medium is passed, for the separation of the biomolecules, over a chromatography column the packing of which is formed substantially from the clay material.

10. Method according to one of the previous claims, wherein after the separation of the purified liquid medium the biomolecules bound to the clay material are eluted with an eluent.

11. Method according to claim 10, wherein the eluent has a different pH from the liquid medium.

12. Method according to one of the previous claims, wherein the biomolecule is a protein.

13. Method according to one of the previous claims, wherein the liquid medium is an aqueous solution or suspension.

14. Method according to one of the previous claims, wherein the clay material is used mixed with a further adsorption material.

15. Method according to claim 14, wherein the further adsorption material is selected from the group which is formed by silica gel, cellulose and polyvinylpyrrolidone.

16. Method according to claim 14 or 15, wherein the clay material and the further absorbent are present in a ratio of between 1:10 and 10:1.

17. Method according to one of the previous claims, wherein the clay material has a water content of more than 30 wit.-%.

18. Method according to one of the previous claims, wherein the clay material has a heavy-metal leaching of less than 1.5 ppm/100 g arsenic and less than 10 ppm/100 g lead.

19. Method according to one of the previous claims, wherein the liquid medium is a waste water which contains organic or microbial impurities.

20. Method according to one of the previous claims, wherein the liquid medium is beer.

21. Method according to claim 1B, wherein the beer is treated with the clay material by adding the clay material to the beer, with the result that a suspension is obtained and the clay material is then separated from the suspension by a precoat/cake Filtration.

22. Method according to claim 19, wherein the clay material is mixed with silica gel as further adsorbent.

**Revendications**

1. Procédé de séparation de biomolécules d'un milieu liquide,

- un milieu liquide contenant des biomolécules étant mis à disposition,
- le milieu liquide étant traité avec une matière argileuse qui présente

- une surface spécifique supérieure à 150 m$^2$/g,
- un volume poreux supérieur à 0,35ml/g,
- une capacité d'échange cationique supérieure à 40 meq/100 g,
- un volume de sédiments dans l'eau inférieur à 15 ml/2 g, et

- le milieu liquide purifié étant séparé de la matière argileuse.

2. Procédé selon la revendication 1, en rapport à la matière argileuse exempte d'eau (atro), la matière argileuse présentant une teneur en $Al_2O_3$ inférieure à 11 % en poids.

3. Procédé selon la revendication 1 ou 2, en rapport à la matière argileuse exempte d'eau (atro), la matière argileuse présentant une teneur en $Si_2O_3$ supérieure à 65 % en poids.

4. Procédé selon l'une quelconque des revendications précédentes, au moins 40 % du volume poreux de la matière argileuse étant mis à disposition par des pores présentant un diamètre de pores d'au moins 14 nm.

5. Procédé selon l'une quelconque des revendications précédentes, après repos à température ambiante pendant trois jours dans de l'eau, le volume de sédiments de la matière argileuse étant inférieur à 15 ml/2g, de préférence inférieur à 10 ml/2 g.

6. Procédé selon l'une quelconque des revendications précédentes, la matière argileuse ayant été activée par traitement de surface à l'acide.

7. Procédé selon l'une quelconque des revendications précédentes, pour la séparation des biomolécules, le milieu liquide étant dirigé à travers un bloc de filtres qui est au moins formé proportionnellement dans la matière argileuse.

8. Procédé selon la revendication 7, le bloc de filtres étant fabriqué par une filtration à couches.

9. Procédé selon l'une quelconque des revendications précédentes, pour la séparation des biomolécules, le milieu liquide étant dirigé à travers une colonne chromatographique, dont le bloc est formé essentiellement dans la matière argileuse.

**10.** Procédé selon l'une quelconque des revendications précédentes, après la séparation du milieu liquide purifié, les biomolécules liées à la matière argileuse étant éluées avec un produit d'élution.

**11.** Procédé selon la revendication 10, le produit d'élution présentant une valeur pH différente de celle du milieu liquide.

**12.** Procédé selon l'une quelconque des revendications précédentes, la biomolécule étant une protéine.

**13.** Procédé selon l'une quelconque des revendications précédentes, le milieu liquide étant une solution ou une suspension aqueuse.

**14.** Procédé selon l'une quelconque des revendications précédentes, la matière argileuse étant utilisée avec une autre matière d'adsorption.

**15.** Procédé selon la revendication 14, l'autre matière d'adsorption étant choisie dans le groupe qui est formé par le gel de silice, la cellulose et la polyvinylpyrrolidone.

**16.** Procédé selon la revendication 14 ou 15, la matière argileuse et l'autre matière d'adsorption se présentant dans une proportion comprise entre 1:10 et 10:1.

**17.** Procédé selon l'une quelconque des revendications précédentes, la matière argileuse présentant une teneur en eau supérieure à 30 % en poids.

**18.** Procédé selon l'une quelconque des revendications précédentes, la matière argileuse présentant une lixiviation de métaux lourds inférieure à 1,5 ppm/100 g d'arsenic et inférieure à 10 ppm/100 g de plomb.

**19.** Procédé selon l'une quelconque des revendications précédentes, le milieu liquide étant un effluent qui contient des impuretés organiques ou microbiennes.

**20.** Procédé selon l'une quelconque des revendications précédentes, le milieu liquide étant de la bière.

**21.** Procédé selon la revendication 18, la bière étant traitée avec la matière argileuse, en ce que la matière argileuse est rajoutée à la bière, de sorte à obtenir une suspension et en ce que la matière argileuse est ensuite séparée de la suspension par une filtration à couches/à gâteaux.

**22.** Procédé selon la revendication 19, la matière argileuse étant mélangée à du gel de silice en tant qu'autre matière d'adsorption.

**Eiweißadsorption**

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2004103552 A
- DE 1160812
- DE 1717084 A
- DE 1717085 B
- DE 1517888 A
- DE 1642767 B
- DE 10237518 A1
- EP 0566260 B1
- DE 4140746 A1
- US 5256300 A
- US 4458030 A
- WO 0112570 A1
- WO 9902256 A
- US 5008226 A
- US 5869415 A

**In der Beschreibung aufgeführte Nicht-Patentliteratur**

- **C. Buttersack ; K. Nowikow ; A. Schaper ; K. Buchholz.** *Zuckerind.,* 1994, vol. 119 (4), 284-291
- **E.P Barrett ; L.G. Joyner ; P.P. Haienda.** *J. Am. Chem. Soc.,* 1951, vol. 73, 373
- **M. Bradford.** *Anal. Biochem.,* 1976, vol. 72, 248-254